Europäisches Patentamt

(19) European Patent Office

Office européen des brevets

(11) Veröffentlichungsnummer: **0 195 252**
**B1**

(12) **EUROPÄISCHE PATENTSCHRIFT**

(45) Veröffentlichungstag der Patentschrift: **16.01.91**

(21) Anmeldenummer: **86101987.5**

(22) Anmeldetag: **17.02.86**

(51) Int. Cl.⁵: **B 01 J 23/85,** C 07 C 5/333,
C 07 C 15/46

(54) **Dehydrierungskatalysator und dessen Verwendung.**

(30) Priorität: **21.02.85 DE 3506022**

(43) Veröffentlichungstag der Anmeldung:
**24.09.86 Patentblatt 86/39**

(45) Bekanntmachung des Hinweises auf die
Patenterteilung:
**16.01.91 Patentblatt 91/03**

(84) Benannte Vertragsstaaten:
**BE DE FR GB NL**

(56) Entgegenhaltungen:
**DE-A-2 815 812**
**DE-A-3 132 014**
**FR-A-2 474 486**
**US-A-4 261 864**

(73) Patentinhaber: **BASF Aktiengesellschaft**
**Carl-Bosch-Strasse 38**
**D-6700 Ludwigshafen (DE)**

(72) Erfinder: **Ambach, Eberhard, Dr.**
**Carl-Bosch-Strasse 7**
**D-6703 Limburgerhof (DE)**
Erfinder: **Biffar, Werner, Dr.**
**Fontanesistrasse 11**
**D-6710 Frankenthal (DE)**
Erfinder: **Mross, Wolf Dieter, Dr.**
**Anselm-Feuerbach-Strasse 21**
**D-6710 Frankenthal (DE)**
Erfinder: **Schwarzmann, Matthias, Dr.**
**Carl-Bosch-Strasse 54**
**D-6703 Limburgerhof (DE)**
Erfinder: **Becker, Hans-Juergen, Dr.**
**Bergsteinstrasse 36**
**D-6730 Neustadt (DE)**
Erfinder: **Kuessner, Klaus, Dr.**
**Weidenstrasse 3**
**D-6710 Frankenthal (DE)**

EP 0 195 252 B1

Courier Press, Leamington Spa, England.

**Beschreibung**

Die Erfindung betrifft einen Dehydrierungskatalysator, der eine Eisenverbindung, eine Kaliumverbindung, eine Wolframverbindung und mindestens ein Oxid oder Karbonat von Sc, Y, La, Cr, Al und/oder den seltenen Erden, enthält.

Zum Stand der Technik nennen wir:

(1) Kunststoff-Handbuch, Band V, Polystyrol, Carl Hanser-Verlag, S. 39—47 (1969)
(2) US—PS 4 144 197
(3) DE—OS 2 544 185
(4) DE—OS 3 132 014
(5) US—PS 3 361 683
(6) US—PS 3 084 125
(7) DE—OS 2 815 812
(8) US—PS 3 223 743
(9) DE—OS 2 406 280
(10) US—PS 2 990 432

Olefine, insbesondere Butadien und Vinylbenzole sind wichtige Ausgangsprodukte für die Herstellung von Kuststoffen, synthetischen Harzen und Kautschukarten.

Im allgemeinen werden sie bei den technisch ausgeübten Verfahren durch Überleiten von teilgesättigten Kohlenwasserstoffen, z.B. Ethylbenzol oder Buten, zusammen mit Wasserdampf über einen Festbettkatalysator bei Temperaturen zwischen 450 und 700°C dehydriert. Das Verfahren kann isotherm ode adiabatisch ausgeführt werden (vgl. 1).

Als Katalysatoren werden Eisenoxide, promotiert mit oxidbildenden Alkalisalzen und weiteren selektivitätssteigernden und strukturstabilisierenden Metallverbindungen verwendet.

In den großtechnischen Dehydrierverfahren werden große Stoffmengen umgesetzt. Wegen den großen Mengen ist selbst eine prozentual kleine Verbesserung der Ausbeuten von wirtschaftlichem Interesse. Die Ausbeute wird zum einen von der Selektivität, zum anderen von der Aktivität des Katalysators bestimmt. Eine hohe Aktivität hat einen hohen Umsatz zur Folge. Die Ausbeute ist als Produkt "Selektivität × Umsatz × 1/100" definiert. Günstig ist demnach ein Katalysator, welche sowohl eine hohe Selektivität als auch eine hohe Aktivität besitzt. Im allgemeinen sind diese Eigenschaften einander gegenläufig, d.h. ein selektiver Katalysator ist meist wenig aktiv und umgekehrt. Die Zugabe von selektivitätssteigernden Promotoren führt oft zu einer Verringerung der Aktivität.

Der Aktivitätsverlust kann in manchen Fällen durch Zugabe eines weiteren Promotors wieder ausgeglichen werden. Nach den Angaben in (5) und (6) wird die Selektivität von Eisenoxid-Chromoxid-Kaliumoxid-Katalysatoren durch den Zusatz von Vanadinpentoxid verbessert. Gemäß (7) kann der Aktivitätsverlust durch gleichzeitige Zugabe eines Oxids von Al, Cd, Cu, Mg, Mn, Ni, V, Zn oder einem Seltenen Erdmetalloxid oder deren Gemischen wieder ausgeglichen werden. Gemäß (4) wird die Desaktivierung der Katalysatoren durch den Zusatz einer Lithiumverbindung vermieden. In (2) werden Katalysatoren mit Vanadinoxid, Wolframoxid, Ceroxid und Kobaltoxid als Promotoren beschrieben.

Für die Lebensdauer eines Katalysators ist die Zugabe von strukturstabilisierenden Komponenten entscheidend. Diese Strukturstabilisatoren sind oft gleichzeitig Promotoren, sie beeninflussen Selektivität und Aktivität der Katalysatoren. In (8) wird die Kombinaton Chromoxid/Ceroxid und in (9) die von Molybdänoxid/Ceroxid als Strukturstabilisator und Promotor vorgeschlagen.

Zur Erhöhung der physikalischen Festigkeit und zur Verbesserung der Selektivität und der Aktivität wird in (10) hydraulischer Zement, wie Porlandzement, verwendet.

Ausgehend von diesem Stand der Technik bestand die Aufgabe einen Katalysator für die Dehydrierung von alkylierten Aromaten, speziell von Ethylbenzol zu Styrol, bei gleichzeitig höherer Aktivität und Selektivität vorzuschlagen, der sich unter den Reaktionsbedingungen selbst regeneriert.

Die Lösung diese Aufgabe erfolgt durch einen Katalysator gemäß Patentanspruch 1.

Die Erfindung betrifft daher einen Dehydrierungskatalysator, bestehend aus den Komponenten

a) 40 bis 90 Gew.% einer Eisenverbindung, berechnet als $Fe_2O_3$,
b) 5 bis 40 Gew.% einer Kaliumverbindung, berechnet als stabilstes Oxid,
c) 1 bis 15 Gew.% einer Wolframverbindung, berechnet als $WO_3$, und
d) 0,1 bis 15 Gew.% mindestens einer Verbindung von Sc, Y, La, Cr, Al und/oder den Seltenen Erden, berechnet als Oxid,

wobei der Katalysator zusätzlich

e) 0,1 bis 15 Gew.% mindestens einer Verbindung von Zn, Cd und/oder den Erdalkalimetallen, berechnet als Oxid, aufweist.

Nachstehend wird der Aufbau des erfindungsgemäßen Katalysators aus Komponenten a) bis e) und seine Herstellung beschrieben.

Die Komponenten a) bis e) machen in der Regel 100% aus. Zu seiner Herstellung können Verformungs-Hilfsmittel, wie Graphit, Cellulose, Stärke, Bentonit, Portlandzement etc. benutzt werden, wobei organische Zusätze bei einer Calcinierung des Katalysators abbrennen können und somit die Zusammensetzung des Katalysators vorteilhaft nicht wesentlich beeninflussen. Die nachstehend genannte Zusammensetzung bezieht sich immer auf die "oxidische Form" vor der Benutzung und frei von Hilfsmitteln.

EP 0 195 252 B1

Der erfindungsgemäße Katalysator besteht vorzugsweise aus;
a) 50 bis 90 Gew.%, insbesondere 60 bis 80 Gew.%, einer Eisenverbindung, berechnet als $Fe_2O_3$,
b) 10 bis 20 Gew.%, einer Kaliumverbindung, berechnet als stabilstes Oxid,
c) 2 bis 9 Gew.%, einer Wolframverbindung, berechnet als $WO_3$,
d) 0,5 bis 12 Gew.%, insbesondere 1 bis 9 Gew.%, mindestens einer Oxids oder Carbonats von Sc, Y, La, Cr, Al und/oder den Seltenen Erden, berechnet als Oxid, insbesondere 3 bis 6.5 Gew.% einer Verbindung von Cr oder Cer, berechnet als Oxid, und
e) 0,5 bis 12 Gew.%, insbesondere 5 bis 10 Gew.%, eines Oxids oder Carbonats von Zn, Cd und/oder den Erdalkalimetallen, berechnet als Oxid, insbesondere 6 bis 10 Gew.% mindestens eines Carbonats von Ba oder Ca, berechnet als Oxid.

Komponente a)

Als Eisenverbindung können verschiedenen Eisenoxide oder Eisenoxidhydrate verwendet werden. Bevorzugt verwendet man freinkristalline, gelbe ($\alpha$-FeOOH), rote ($\alpha$-$Fe_2O_3$) oder schwarze ($Fe_3O_4$) Pigmente.

Komponente b)

Als Kaliumverbindung sind das Oxid, Hydroxid, Carbonat, Hydrogencarbonat oder alle Kaliumsalze, welche beim Erhitzen in das Oxid übergehen, geeignet. Im allgemeinen wird Kaliumcarbonat bevorzugt.

Komponente c)

Die Wolframverbindung wird vorzugsweise in Form von Wolframoxid oder einer Verbindung, welche sich beim Calcinieren in das Oxid zersetzt, z.B. als Wolframsäure, zugegeben.

Komponente d)

Sc, Y, La, Cr, Al und/oder die Seltenen Erden werden in Form ihrer Verbindungen, z.B. der Nitrate, Hydroxide, vorzugsweise aber als Oxide oder Carbonate eingesetzt. Von den Seltenen Erden werden dabei Cer, Praseodym und Europium bevorzugt.

Komponente e)

Die Zn-, Cd- und/oder Erdalkaliverbindung wird vorzugsweise als Oxid oder Carbonat zugesetzt. Besonders bevorzugt wird Calciumcarbonat.

Die Herstellung des erfindungsgemäßen Katalysators kann auf verschiedene Weise erfolgen. Die feingepulverten Bestandteile können in Wasser suspendiert und sprühgetrocknet oder, was einfacher ist, nur trocken gemischt werden.

Das trockene Pulver wird anschließend gegebenenfalls nach Zugabe eines geeigneten Verformungs-Hilfsmittels entweder zu mechanisch stabilen Formkörpern tablettiert oder unter Zugabe von Wasser zu einer pastösen Masse angeteigt und zu Strängen extrudiert. Die Extrudate werden getrocknet und können gegebenenalls bei Temperaturen von 300 bis 900°C calciniert werden.

Beispiel für die Herstellung eines erfindungsgemäßen Katalysators folgender Zusammensetzung:

| Stoff | Gewichtsteile |
|---|---|
| $\alpha$-FeOOH | 1 000 |
| $K_2CO_3$ | 357 |
| $WO_3$ | 62 |
| $Ce_2(CO_3)_3$ | 111 |
| $CaCO_3$ | 208 |

Die oben angegebenen pulverförmigen Komponenten werden sorgfältig vermischt und nach Zugabe von 270 ml Wasser 3 h lang geknetet. Die so erhaltene, pastöse Kontaktmasse wird zu Strängen mit einem Durchmesser von 6 mm extrudiert, 1 h lang bei 80°C getrocknet und anschließend 2 h lang bei 400°C calciniert. Alle in der Tabelle aufgeführten weiteren erfidungsgemäßen Katalysatoren 2 bis 20 wurden nach diesem Rezept hergestellt. In der Tabelle sind für alle Katalysatoren Zusammensetzung und Selektivität angegeben. Zum Vergleich ist in der Tabelle unter Nummer 21 das Ergebnis eines Versuchs aufgeführt, bei dem ein Kontakt der Zusammensetzung 77,9% $Fe_2O_3$, 12,6% $K_2O$, 2,3% $WO_3$, 2,7% $Ce_2O_3$, 1,2% $Cr_2O_3$ und 0,3% CoO gemäß dem Beispiel in Tabelle IX der DE—AS 28 15 874 verwendet wurde.

Testvorschrift für die erfindungsgemäßen Katalysatoren zur beispielhaften Dehydrierung von Ethylbenzol zu Styrol:

Die Katalysatoren 1 bis 20 werden auf eine Körnung von 0,5 bis 1 mm zerkleinert. Von diesen

3

Kontaktmassen werden jeweils 20 ml in einen Reaktor mit 6 mm Innendurchmesser eingefüllt. Pro Stunde werden 20 ml Wasser und 10 ml Ethylbenzol in den Reaktor dosiert. Die Temperatur des zur Reaktorheizung verwendeten Salzbades wird jeweils so eingestellt, daß die organische Phase des Reaktoraustrages 50% Styrol enthält. Nach 6 Tagen wird eine Gesamtanalyse der organischen Phase und des Abgases durchgeführt und die Selektivität des Katalysators errechnet.

## Tabelle

EP 0 195 252 B1

| Kataly-sator Nr. | a) | Komponenten des Katalysators in Gewichtsteilen * b) | c) | d) | e) |
|---|---|---|---|---|---|
| 1 | 100,0 (73,7) FeOOH; | 20,0 (11,2) $K_2CO_3$; | 6,2 (5,1) $WO_3$; | 4,0 (3,3) $CrO_3$: | 10,5 (6,7) $BaCO_3$ |
| 2 | 100,0 (74,8) FeOOH; | 20,0 (11,3) $K_2CO_3$; | 6,2 (5,2) $WO_3$; | 2,3 (1,9) $Al_2O_3$: | 10,5 (6,8) $BaCO_3$; |
| 3 | 100,0 (71,5) FeOOH; | 20,0 (10,8) $K_2CO_3$; | 6,2 (4,9) $WO_3$; | 11,1 (6,3) $Ce_2(CO_3)_3$: | 10,5 (6,5) $BaCO_3$; |
| 4 | 100,0 (65,9) FeOOH; | 35,7 (17,8) $K_2CO_3$; | 6,2 (4,5) $WO_3$; | 11,1 (5,8) $Ce_2(CO_3)_3$: | 10,5 (6,0) $BaCO_3$; |
| 5 | 100,0 (63,6) FeOOH; | 35,7 (17,2) $K_2CO_3$; | 6,2 (4,4) $WO_3$; | 11,1 (5,6) $Ce_2(CO_3)_3$: | 5,8 (3,2) $BaCO_3$; 15,0 (5,9) $CaCO_3$; |
| 6 | 100,0 (64,2) FeOOH; | 35,7 (17,4) $K_2CO_3$; | 6,2 (4,4) $WO_3$; | 11,1 (5,7) $Ce_2(CO_3)_3$: | 20,8 (8,3) $CaCO_3$; |
| 7 | 100,0 (69,6) FeOOH; | 20,0 (10,5) $K_2CO_3$; | 6,2 (4,8) $WO_3$; | 11,1 (6,1) $Ce_2(CO_3)_3$: | 20,8 (9,0) $CaCO_3$; |
| 8 | 100,0 (76,4) FeOOH; | 20,0 (11,6) $K_2CO_3$; | 6,2 (5,3) $WO_3$; | 11,1 (6,7) $Ce_2(CO_3)_3$: | |
| 9 | 100,0 (66,2) FeOOH; | 35,7 (17,9) $K_2CO_3$; | 6,2 (4,6) $WO_3$; | 3,6 (2,7) $Sc_2O_3$: | 20,8 (8,6) $CaCO_3$; |
| 10 | 100,0 (66,0) FeOOH; | 35,7 (17,9) $K_2CO_3$; | 6,2 (4,6) $WO_3$; | 4,0 (2,9) $Y_2O_3$: | 20,8 (8,6) $CaCO_3$; |
| 11 | 100,0 (66,0) FeOOH; | 35,7 (17,9) $K_2CO_3$; | 6,2 (4,6) $WO_3$; | 4,0 (2,9) $CrO_3$: | 20,8 (8,6) $CaCO_3$; |
| 12 | 100,0 (65,6) FeOOH; | 35,7 (17,8) $K_2CO_3$; | 3,1 (2,3) $WO_3$; | 11,1 (5,8) $Ce_2(CO_3)_3$: | 20,8 (8,5) $CaCO_3$; |
| 13 | 100,0 (61,5) FeOOH; | 35,7 (16,6) $K_2CO_3$; | 12,4 (8,5) $WO_3$; | 11,1 (5,4) $Ce_2(CO_3)_3$: | 20,8 (8,0) $CaCO_3$; |
| 14 | 100,0 (65,9) FeOOH; | 35,7 (17,9) $K_2CO_3$; | 6,2 (4,6) $WO_3$; | 6,0 (3,1) $Ce_2(CO_3)_3$: | 20,8 (8,5) $CaCO_3$; |
| 15 | 100,0 (62,6) FeOOH; | 35,7 (17,0) $K_2CO_3$; | 6,2 (4,3) $WO_3$; | 16,1 (8,0) $Ce_2(CO_3)_3$: | 20,8 (8,1) $CaCO_3$; |
| 16 | 100,0 (64,3) FeOOH; | 35,7 (17,4) $K_2CO_3$; | 6,2 (4,4) $WO_3$; | 11,1 (5,6) $La_2(CO_3)_3$: | 20,8 (8,3) $CaCO_3$; |
| 17 | 100,0 (65,9) FeOOH; | 35,7 (17,9) $K_2CO_3$; | 6,2 (4,5) $WO_3$; | 11,1 (5,8) $Ce_2(CO_3)_3$: | 12,3 (5,9) $ZnCO_3$; |
| 18 | 100,0 (64,2) FeOOH; | 35,7 (17,4) $K_2CO_3$; | 6,2 (4,4) $WO_3$; | 11,1 (5,7) $Pr_2(CO_3)_3$: | 20,8 (8,3) $CaCO_3$; |
| 19 | 100,0 (65,9) FeOOH; | 35,7 (17,9) $K_2CO_3$; | 6,2 (4,5) $WO_3$; | 11,1 (5,8) $La_2(CO_3)_3$: | 12,3 (5,9) $ZnCO_3$; |
| 20 | 100,0 (64,5) FeOOH; | 35,7 (17,4) $K_2CO_3$; | 6,2 (4,4) $WO_3$; | 11,1 (5,7) $Ce_2(CO_3)_3$: | 15,0 (8,0) $CdCO_3$; |
| 21 | 100,0 (77,9) FeOOH; | 21,3 (12,6) $K_2CO_3$; | 2,7 (2,3) $WO_3$; | 4,4 (2,7) $Ce_2(CO_3)_3$: | 1,4 (1,2) $Cr_2O_3$; 0,4 (0,3) $Co(OH)_2$; |

*Alle Werte in Klammern sind Gew.% bezogen auf die Summe der Komponenten a) bis e) = 100 unter Annahme der stabilsten Oxide. z.B. $Fe_2O_3$; $K_2O$; $WO_3$; $Ce_2O_3$; $La_2O_3$; $Pr_2O_3$, BaO, CaO, ZnO und CdO.

| Katalysator Nr. | Testung Reaktionstemperatur (°C) | Selektivität (%) |
|---|---|---|
| 1 | 619 | 95,7 |
| 2 | 615 | 95,4 |
| 3 | 614 | 95,9 |
| 4 | 598 | 95,3 |
| 5 | 584 | 97,2 |
| 6 | 579 | 97,3 |
| 7 | 595 | 96,8 |
| 8 | 598 | 96,0 |
| 9 | 608 | 95,2 |
| 10 | 605 | 95,9 |
| 11 | 610 | 95,5 |
| 12 | 590 | 95,9 |
| 13 | 593 | 95,6 |
| 14 | 589 | 96,0 |
| 15 | 592 | 96,8 |
| 16 | 608 | 95,3 |
| 17 | 596 | 96,0 |
| 18 | 585 | 95,2 |
| 19 | 611 | 95,1 |
| 20 | 601 | 96,5 |
| 21 | 620 | 94,8 |

**Patentansprüche**

1. Dehydrierungskatalysator, bestehend aus den Komponenten
a) 40 bis 90 Gew.% einer Eisenverbindung, berechnet als $Fe_2O_3$,
b) 5 bis 40 Gew.% einer Kaliumverbindung, berechnet als stabilstes Oxid,
c) 1 bis 15 Gew.% einer Wolframverbindung, berechnet als $WO_3$, und
d) 0,1 bis 15 Gew.% mindestens einer Verbindung von Sc, Y, La, Cr, Al und/oder den Seltenen Erden, berechnet als Oxid,
und gegebenenfalls Verformungshilfsmitteln, dadurch gekennzeichnet, daß der Katalysator zusätzlich
e) 0,1 bis 15 Gew.% mindestens einer Verbindung von Zn, Cd und/oder den Erdalkalimetallen, bererchnet als Oxid, aufweist.

2. Dehydrierungskatalysator nach Anspruch 1, bestehend aus den Komponenten
a) 50 bis 90 Gew.% einer Eisenverbindung, berechnet als $Fe_2O_3$,
b) 10 bis 20 Gew.% einer Kaliumverbindung, berechnet als stabilstes Oxid,
c) 2 bis 9 Gew.% einer Wolframverbindung, berechnet als $WO_3$, und
d) 0,5 bis 12 Gew.% mindestens einer Verbindung von Sc, Y, La, Cr, Al und/oder den Seltenen Erden, berechnet als Oxid,
und gegenbenefalls Verformungshilfsmitteln, dadurch gekennzeichnet, daß der Katalysator zusätzlich
e) 0,5 bis 12 Gew.%, mindestens einer Verbindung von Zn, Cd, Ca, Sr oder Ba oder von Gemischen aus diesen, berechnet als Oxid, aufweist.

3. Dehydrierungskatalysator nach Anspruch 1, bestehend aus den Komponenten
a) 60 bis 80 Gew.% einer Eisenverbindung, berechnet als $Fe_2O_3$,
b) 10 bis 20 Gew.% einer Kaliumverbindung, berechnet als stabilstes Oxid,
c) 2 bis 9 Gew.% einer Wolframverbindung, berechnet als $WO_3$, und
d) 1 bis 9 Gew.% mindestens einer Verbindung von Sc, Y, La, Cr, Al oder den Seltenen Erden, berechnet als Oxid,

und gegebenenfalls Verformungshilfsmitteln, dadurch gekennzeichnet, daß der Katalysator zusätzlich

e) 5 bis 10 Gew.% mindestens einer Verbindung von Zn, Cd, Ca, Sr oder Ba oder Gemischen von Ba und Ca, berechnet als Oxid, aufweist.

4. Dehydrierungskatalysator nach Anspruch 1, bestehend aus den Komponenten

a) 60 bis 80 Gew.% einer Eisenverbindung, berechnet als $Fe_2O_3$,

b) 10 bis 20 Gew.% einer Kaliumverbindung, berechnet als stabilstes Oxid,

c) 2 bis 9 Gew.% einer Wolframverbindung, berechnet als $WO_3$, und

d) 3 bis 6,5 Gew.% einer Verbindung von Cr oder Cer, berechnet als Oxid und gegebenenfalls Verformungshilfsmitteln, dadurch gekennzeichnet, daß der Katalysator zusätzlich

e) 6 bis 10 Gew.% mindestens eines Carbonats von Barium oder Calcium oder von Gemischen aus diesen, berechnet, als Oxid, aufweist.

5. Verwendung des Katalysators gemäß Anspruch 1 zur Dehydrierung von Ethylbenzol zu Styrol.

## Revendications

1. Catalyseur de déshydrogénation comprenant les constituants

a) 40 à 90% en poids d'un composé de fer, calculé en $Fe_2O_3$

b) 5 à 40% en poids d'un composé de potassium, calculé en oxyde le plus stable

c) 1 à 15% en poids d'un composé de tungstène, calculé en $WO_3$, et

d) 0,1 à 15% en poids d'au moins un composé de Sc, Y, La, Cr, Al et/ou de terres rares, calculé en oxyde,

et éventuellement des adjuvants de mise en forme caractérisé en ce que le catalyseur a en outre

e) de 0,1 à 15% en poids d'au moins un composé de Zn, Cd et/ou de métaux alcalino-terreux, calculé en oxyde.

2. Catalyseur de déshydrogénation selon la revendication 1, comprenant les constituants

a) 50 à 90% en poids d'un composé de fer, calculé en $Fe_2O_3$

b) 10 à 20% en poids d'un composé de potassium, calculé en oxyde le plus stable

c) 2 à 9% en poids d'un composé de tungstène, calculé en $WO_3$, et

d) 0,5 à 12% en poids d'au moins un composé de Sc, Y, La, Cr, Al et/ou de terres rares, calculé en oxyde,

et éventuellement des adjuvants de mise en forme, caractérisé en ce que le catalyseur a en outre

e) de 0,5 à 12% en poids d'au moins un composé de Zn, Cd, Ca, Sr ou Ba ou de mélanges de ceux-ci, calculé en oxyde.

3. Catalyseur de déshydrogénation selon la revendication 1, comprenant les constituants

a) 60 à 80% en poids d'un composé de fer, calculé en $Fe_2O_3$

b) 10 à 20% en poids d'un composé de potassium, calculé en oxyde le plus stable,

c) 2 à 9% en poids d'un composé de tungstène, calculé en $WO_3$, et

d) 1 à 9% en poids d'au moins un composé de Sc, Y, La, Cr, Al et/ou de terres rares, calculé en oxyde,

et éventuellement des adjuvants de mise en forme, caractérisé en ce que le catalyseur a en outre

e) de 5 à 10% en poids d'au moins un composé de Zn, Cd, Ca, Sr ou Ba ou de mélanges de Ca et Ba, calculé en oxyde.

4. Catalyseur de déshydrogénation selon la revendication 1, comprenant les constituants

a) 60 à 90% en poids d'un composé de fer, calculé en $Fe_2O_3$

b) 10 à 20% en poids d'un composé de potassium, calculé en oxyde le plus stable

c) 2 à 9% en poids d'un composé de tungstène, calculé en $WO_3$, et

d) 3 à 6.5% en poids d'un composé de Cr ou Ce, calculé en oxyde, et éventuellement des adjuvants de mise en forme caractérisé en ce que le catalyseur a en outre

e) de 6 à 10% en poids d'au moins un carbonate de baryum ou de calcium ou de melanges de ceux-ci, calculé en oxyde.

5. Utilisation de catalyseur selon la revendication 1 pour la déshydrogénation de l'éthylbenzène en styrène.

## Claims

1. A dehydrogenation catalyst comprising

a) 40—90% by weight of an iron compound, calculated as $Fe_2O_3$,

b) 5—40% by weight of a potassium compound, calculated as the most stable oxide,

c) 1—15% by weight of a tungsten compound, calculated as $WO_3$, and

d) 0.1—15% by weight of at least one compound of Sc, Y, La, Cr, Al or of a rare earth, calculated as oxide,

and optionally a demolding assistant, additionally containing

e) 0.1—15% by weight of at least one compound of Zn, Cd or of an alkaline earth metal, calculated as oxide.

2. A dehydrogenation catalyst according to claim 1, comprising

a) 50—90% by weight of an iron compound, calculated as $Fe_2O_3$,

7

b) 10—20% by weight of a potassium compound, calculated as the most stable oxide,

c) 2—9% by weight of a tungsten compound, calculated as $WO_3$, and

d) 0.5—12% by weight of at least one compound of Sc, Y, La, Cr, Al or of a rare earth, calculated as oxide,

and optionally a demolding assistant, additionally containing

e) 0.5—12% by weight of at least one compound of Zn, Cd, Ca, Sr or Ba or of a mixture thereof, calculated as oxide.

3. A dehydrogenation catalyst according to claim 1, comprising

a) 60—80% by weight of an iron compound, calculated as $Fe_2O_3$,

b) 10—20% by weight of a potassium compound, calculated as the most stable oxide,

c) 2—9% by weight of a tungsten compound, calculated as $WO_3$, and

d) 1—9% by weight of at least one compound of Sc, Y, La, Cr, Al or of a rare earth, calculated as oxide,

and optionally a demolding assistant, additionally containing

e) 5—10% by weight of at least one compound of Zn, Cd, Ca, Sr or Br or of a mixture thereof, calculated as oxide.

4. A dehydrogenation catalyst according to claim 1, comprising

a) 60—80% by weight of an iron compound, calculated as $Fe_2O_3$,

b) 10—20% by weight of a potassium compound, calculated as the most stable oxide,

c) 2—9% by weight of a tungsten compound, calculated as $WO_3$, and

d) 3—6.5% by weight of a compound of Cr or cerium, calculated as oxide, and optionally a demolding assistant, additionally containing

e) 6—10% by weight of at least one compound of barium or calcium or of a mixture thereof, calculated as oxide.

5. A method of using a catalyst as claimed in claim 1 for dehydrogenating ethylbenzene to styrene.